# EUROPEAN PATENT APPLICATION

(11) **EP 2 620 505 A1**
(43) Date of publication of application: **31.07.2013**
(21) Application number: 12152372.4
(22) Date of filing: 25.01.2012
(51) Int. Cl.: C12P 7/64, C12P 19/12

(54) **Maltose and maltose oligomer esterification process**

(71) Applicant: DSM IP Assets B.V., 6411 TE Heerlen (NL)
(72) Inventor: The designation of the inventor has not yet been filed
(74) Representative: Schwander, Kuno

(57) **Abstract**

The present invention relates to a green and improved process for the lipase mediated esterification of maltose or a maltose oligomer with a medium chain polyunsaturated fatty acid comprising the steps of incubating a maltose oligomer with a polyunsaturated fatty acid in the presence of a lipase, a binary solvent and an activated molecular sieve. The invention furthermore relates to the use of said process for the production of mono-linoleyl-α-D-Maltose with a ratio 6'-O-linoleyl-α-D-maltose: 6-O-linoleyl-α-D-maltose comprised between 62:38 and 54:46.

## Description

The present invention relates to a green and improved process for the lipase mediated esterification of maltose or a maltose oligomer with a medium chain polyunsaturated fatty acid comprising the steps of incubating a maltose oligomer with a polyunsaturated fatty acid in the presence of a lipase, a binary solvent and an activated molecular sieve. The invention furthermore relates to the use of said process for the production of mono-linoleyl-α-D-Maltose with a ratio 6'-O-linoleyl-α-D-maltose: 6-O-linoleyl-α-D-maltose comprised between 62:38 and 54:46.

Sugar fatty acid esters are often used as surfactants in different industries. There is an increasing demand in the industry for semi-natural products or for products produced by green chemistry. Therefore, the chemical industry is investing significant efforts to develop green chemistry processes in order to replace old chemical processes. Green chemistry, also called sustainable chemistry, is a philosophy of chemical research and engineering that encourages the design of products and processes that minimize the use and generation of hazardous substances. Whereas environmental chemistry is the chemistry of the natural environment, and of pollutant chemicals in nature, green chemistry seeks to reduce and prevent pollution at its source. As a chemical philosophy, green chemistry applies to organic chemistry, inorganic chemistry, biochemistry, analytical chemistry, and even physical chemistry. While green chemistry seems to focus on industrial applications, it does apply to any chemistry choice.

The eco-efficient lipase mediated/catalyzed synthesis of sugar fatty acid esters from renewable commodities is an important issue in white biotechnology. Eco-efficient solvents ensure sustainable bio enzymatic conversions and synthesis.

Monosaccharide esterification with fatty acids is a very well established reaction known since the eighties and results in very efficient conversion yields. In contrast, lipase mediated esterification of maltose or maltose oligomers with medium chain polyunsaturated fatty acids, remains an issue in the industry, since efficient conversions have not been reported in the literature so far.

Surprisingly, it has been found that the process according to the present invention delivers much higher conversion yields of the ester products when compared to processes of the prior art. Moreover, the process can be considered an eco-efficient process according to the green chemistry definitions since it uses green organic solvents and/or ionic liquid solvents in a binary solvent system. Finally, this process also uniquely produces regio-selective esters of maltose of which the 6'-O-linoleyl-α-D-maltose is most abundant.

Thus, the present invention provides a green process for the lipase mediated esterification of maltose or a maltose oligomer with a medium chain polyunsaturated fatty acid, wherein said process comprising the steps of:
(a) incubating a medium chain polyunsaturated fatty acid and maltose or a maltose oligomer in the presence of a lipase, a binary solvent and an activated molecular sieve 3Å in a sealed container under agitation for 2 to 7 days at a temperature of 30 to 80°C,
(b) filtering off the lipase and activated molecular sieve,
wherein the binary solvent primarily consists of any mixture of 2 solvents selected from green organic solvents and ionic liquids.

Esterification is the general name for a chemical reaction in which two reactants (typically an alcohol and an acid) form an ester as the reaction product. In the present case the alcohol is the primary alcohol function in position 6 of the sugar moity, while the carboxylic acid function is provided by the medium chain polyunsaturated fatty acid.

An ionic liquid is a salt in the liquid state. In some contexts, the term has been restricted to salts whose melting point is below some arbitrary temperature, such as 100 °C (212 °F). While ordinary liquids such as water and gasoline are predominantly made of electrically neutral molecules, ionic liquids are largely made of ions and short-lived ion pairs. These substances are variously called liquid electrolytes, ionic melts, ionic fluids, fused salts, liquid salts, or ionic glasses. For the present invention, suitable ionic liquids are water miscible and immiscible ionic liquids, were ionic liquids at room temperature, or melted below a temperature of 60°C.

Moreover, for the present invention, green organic solvents were selected from toluol, DMSO, dioxan, hexane, ter-butylethanol, acetonitril, tetrahydrofurane, acetone, dimethylformamide.

Medium chain polyunsaturated fatty acids are C₁₂ to C₂₄ polyunsaturated fatty acids with any number of unsaturated bonds in any position.

A suitable lipase (EC 3.1.1.3) for the process according to the present invention is selected from commercially available lipases isolated from the following microorganisms: Rhisophus arrhizus, Rhisophus oryzae, Aspergillus oryzae, Mucorjavanicus, Candida rugosa, Candida antartica, Pseudomonas fluorescens, Candida cylindracea, Pseudomonas cepacia. More preferred lipase for all the embodiments according to the present invention is the lipase from P. *cepia,* or C. *antartica.* Most preferred lipase is the C. *antartica* immobilized lipase B from Novozymes (SP 435).

In another embodiment the lipase enzyme is added to the reaction mixture at concentration comprised between 1000 and 3000 units lipase per mmol maltose or maltose oligomer, more preferably between 1300 and 2700 units, most preferably, between 1500 and 2200 units. Lipase units are defined as Propyl laurate Units (PLU) per gram. 1 PLU corresponds to 1 pmol propyl laurate formed per minute per gram enzyme. Wth the exception that for the immobilized lipase from Novozyme, (SP435) the units are defined as tributyrin units (TBU) per gram of enzyme. 1 TBU corresponds to 1 µmol butyric acid released per minute per gram of immobilized enzyme.

The preferred maltose oligomer according to the present invention in all embodiments of the present invention is selected from maltotriose, maltotetrose, and maltopentose. Most preferably, maltose is used in the process according to the present invention.

In yet another embodiment, the medium chain polyunsaturated fatty acid is selected from any of C₁₄ to C₂₂ polyunsaturated fatty acids with any number of unsaturated bonds in any position, preferably, any of C₁₆ to C₂₀ polyunsaturated fatty acids with any number of unsaturated bonds in any position, most preferably, the medium chain polyunsaturated fatty acid is linoleic acid.

In view of the superior esterification yield, the binary solvent according to the present invention primarily consists of any mixture of 2 solvents selected from: acetone, acetonitrile, dimethylformamide, tetrahydrofurane, 1-ethyl-3-methylimidazolium methanesulphonate, 1-methyl-3-pentyl imidazolium hexafluorophosphate, and 1-butyl-1-methylpyrrolidinium hexafluorophosphate.

Most preferably, binary solvents are selected from the following combinations: tetrahydrofurane/dimethylformamide, acetone/dimethylformamide, tetrahydrofurane/1-butyl-1-methylpyrrolidinium hexafluorophosphate, acetone/1-ethyl-3-methylimidazolium methanesulphonate, dimethylformamide/1-butyl-1-methylpyrrolidinium hexafluorophosphate, and acetone/1-butyl-1-methylpyrrolidinium hexafluorophosphate. In this later selection of binary solvents, preferred ratio of the two solvents is comprised between 1:5 and 5:1, more preferred, between 1:2 and 2:1.

In another embodiment, the step a) according to the process of the present invention is performed from 2 to 4 days at a temperature of 40 to 70°C.

In yet another embodiment, the molar ratio maltose or maltose oligomer/medium chain polyunsaturated fatty acid is comprised between 0.1 and 1 and more preferably between 0.4 and 0.8.

In another embodiment, the molecular sieve is added to the reaction mixture at a concentration comprised between 15 and 300 g/litre, preferably between 50 to 200 g/litre.

The filtering of step b) can be performed by any standard filtering method. Preferably, the filtration is performed through a fritted glass funnel (G3), or using a paper filter in order to remove the molecular sieve and the lipase or lipase beads. For analysis of the reaction product, the reaction needs to be diluted in an appropriate solvent such as water, CH₃CN/H₂O/THF (70/20/10 v/v).

In the most preferred embodiment, the present invention provides a process for the lipase mediated esterification of maltose with linoleic acid, wherein said process comprising the steps of:
(a) incubating linoleic acid and maltose in the presence of a *C*. *antartica* lipase enzyme, a binary solvent and an activated molecular sieve 3Å in a sealed container under agitation for 2 to 5 days at a temperature of 50 to 70°C,
(b) filtering off the lipase and activated molecular sieve,
wherein the binary solvent is selected from tetrahydrofurane/dimethylformamide, acetone/dimethylformamide, tetrahydrofurane/1-butyl-1-methylpyrrolidinium hexafluorophosphate, acetone/1-ethyl-3-methylimidazolium methanesulphonate, dimethylformamide/1-butyl-1-methylpyrrolidinium hexafluorophosphate, and acetone/1-butyl-1-methylpyrrolidinium hexafluorophosphate, and wherein the ratio of the two solvents is comprised between 1:5 and 5:1.

We also provide the use of the process according to the present invention, for the production of mono-linoleyl-α-D-Maltose with a ratio 6'-O-linoleyl-α-D-maltose: 6-O-linoleyl-α-D-maltose comprised between 62:38 and 54:46, preferably comprised between 60:40 and 58:44.

The invention is further illustrated by the following example.

### Examples

### Example 1: Esterification of maltose with linoleic acid.

### Material

Substrates: D(+)-maltose monohydrate (4-*O*-α-D-Glucopyranosyl-D-glucose) in 99% purity was bought from Fluka [Buchs, Switzerland]. Linoleic acid ((9*Z*,12*Z*)-octadeca-9,12-diene acid) with a purity of 99% was purchased from Acros organics [Belgium], and an industrial quality of (60-74%) was provided by DSM-Pentapharm [Aesch, Switzerland]. Molecular sieves 3Å [Merck, Darmstadt] were activated before use.

Enzymes: *Candida antarctica* immobilized as Novozyme 435 was purchased from Novozyme [Denmark]. Non immobilized lipases were bought from Fluka [Buchs, Switzerland]: *Candida antarctica Lipase B* (10.9 U/mg), *Pseudomonas cepacia* (40 U/mg), *Candida cylindracea* (3.9 U/mg), *Candida rugosa* (2.9 U/mg), *Rhizopus niveus* (1.7 U/mg), *Pseudomonas fluorescens* (36 U/mg), *Mucor javanicus* (9.9 U/mg), *Aspergillus oryzae* (53.8 U/mg), *Rhizopus oryzae* (58.4 U/mg), *Rhizopus arrhizus* (9.2 U/mg).

Organic solvents: Acetone, and tetrahydrofurane (THF) analytical grade, were from Fluka [Buchs, Switzerland]. Acetonitrile and dimethylformamide (DMF) (99%), anhydrous were from Sigma Aldrich [Buchs, Switzerland].

Ionic liquids: 1-butyl-4-methylpyridinium hexafluorophosphate ([bmpyr][PF₆]), and 1-methyl-3-propylimidazolium hexafluorophosphate ([mpim][PF₆]) were from lolitec [Heilbronn, Germany] while 1-ethyl-3-methylimidazolium methanesulfonate ([emim][MeSO₃]) was purchased from Sigma Aldrich (BASF), [Buchs, Switzerland].

### Method A: Lipase mediated reaction in organic solvents

10 mmol (2.80 g) linoleic acid, 5 mmol (1.80 g) D(+)-maltose monohydrate, 0.80 g activated molecular sieve 3Å, 0.90 g (9000 TBU) Novozyme 435, and 8.0 mL organic solvent were added to a 20.0 mL glass vial, sealed, and subsequently processed in an incubator (INFORS AJ110) at 65°C/350 rpm for 3 days (basic screening conditions in *Methods A-D*).0 The molecular sieve and lipase were filtered off using a fritted glass funnel (G3). The maltose-containing liquid was transferred to a 200.0 mL gauged flask and filled up with distilled water. The maltose content was determined by HPLC.

### Method B: Lipase mediated reaction in ionic liquids

The preparation of the reaction mixtures followed *Method A* and the organic solvent was replaced by 3.0 g ionic liquid. After processing 65°C/350 rpm/ 3 days the reaction mixture was dissolved with 10 ml CH₃CN/H₂O/THF (70/20/10 v/v) and homogenized using an ultrasound bath, 2 min/20°C. The enzyme and molecular sieves were separated off using a paper filter (Schleicher und Schüll), transferred to a 200.0 mL gauged flask and filled up with the aforementioned solvent mixture. The maltose conversion was determined by HPLC.

### Method C: Lipase mediated reaction with ionic liquid coated lipase beads

The transformation followed *Method A* without using a solvent but employing ionic liquid coated lipase. The lipase coating was prepared according to a known procedure. For this purpose 1.0 g ionic liquid and 20.0 mL acetonitrile were homogenized in a 50 ml flask. 1.0 g Novozyme 435 was added and mixed for 20 min with a rotavapor at 22°C/1 bar. Then the auxiliary solvent was removed under reduced pressure at 22°C and the remaining coated beads were stored in a dessiccator over night (15 hours) at room temperature. After processing, the remaining maltose content was determined by HPLC.

### Method D: Lipase mediated reaction in binary solvent mixtures

The reaction mixtures were prepared according to *Method A,B.* Binary solvent mixtures were 1:1 (v:v) mixtures of 4.0 mL organic solvent each, or 3.0 g of ionic liquid. In the case of binary ionic liquids, or an organic solvent/ionic liquid mixture either g or ml quantities were employed. After the transformation aliquots of the mixture were directly injected into an HPLC column to determine maltose conversion.

### Enzyme screening

Reaction mixtures were prepared according to *Method A* using acetone with adapted substrate quantities to fit with 1.5 mL Eppendorf vials. The sealed vials were vertically fixed on a revolving glass cylinder in a hybridization oven, 8 rpm/40°C for 1 to 7 days. Maltose conversion was determined by HPLC.

### HPLC Analysis

Maltose concentrations were directly determined from reaction solutions after dilution in a 200 mL gauged flask by HPLC (Agilent 1100 Series with RID) using an Aminex-H87 column (300 mm x 7.8 mm i.D.) at 35 °C with 5 mM H₂SO₄ as eluent (0.5 mL/min).

### Product isolation

The filtered raw product was separated based on an improved HPLC protocol from *Method A*. The preparative HPLC consisted of a Knauer (Germany) setup with Luna 5u C18(2) column (100Å, 250x15mm, 5 micron) [Phenomenex], and UV₂₀₀ₙₘ detector. H₂O/CH₃CN eluted the isomers, several fractions were obtained and the eluent was removed at 40°C/60 mbar.

### Product characterization

*NMR, MS, and IR analysis:* The maltose linoleic acid ester was dissolved in 0.75 mL d₆-DMSO for NMR analyses: ¹H and ¹³C-NMR, COSY (¹H-¹H correlation), HSQC (¹H-¹³C heteronuclear single quantum coherence), and HMBC (¹H-¹³C heteronuclear multiple bond (³*J*) correlation). All these experiments were conducted on a 400 MHz NMR spectrometer. A supplementary 800 MHz ¹H-NMR spectrum improved hydroxyl proton resolution (both NMR-spectrometers were of the Bruker Avance type). A Varian 320-MS TQ mass spectrometer with electrospray (ESI) unit identified molecular mass peaks. A Nicolet FT-IR Thermo 5700 spectrometer (KBr tablet) was used for carbonyl function analysis.

### Results and Discussion

### Biotransformation in organic solvents

Several organic solvents enabled the maltose acylation with linoleic acid using identical generally applied screening parameters, 65°C/350rpm/3days (*Method A*). The most suited organic solvent DMF allowed 39.7 % maltose conversion. Acetone with 38.1% worked equally well, while THF (33.9%) and acetonitrile (29.7%) performed somewhat lower. The rest of the tested organic solvents showed a descending suitability: tert-butanol > hexane > dioxane > DMSO > toluene (Table 1).

The conversion in liquid linoleic acid without an additional solvent enabled 20.3% maltose conversion. The productivity in organic solvents is in general low and acetone is the most suited solvent. In addition it can be sourced from renewable feedstock through ABE-fermentation.

### Ionic liquids as solvent and enzyme coating

Several ionic liquids in bulk solvent quantities were subjected to screening conditions (*Method B*) and [emim][MeSO₃] clearly excelled (Table 1). There are over a million ionic liquids possible but the choice is limited by their availability. The solution and other physical

properties of all these ionic liquids are certainly diverse but unknown; and even for available ionic liquids, such data is often unavailable. Nevertheless, in a systematic ionic liquid screening some physical solvent parameters can be fixed. Ionic liquids used in the present experiments were either room temperature ionic liquids (RTIL) or melted below 60°C. Secondly also water miscible and immiscible ionic liquids were distinguished. The water miscible [emim][MeSO₃] enabled maltose conversion of 36.8 %, which is equally efficient as well suited organic solvents, like acetone or DMF.

The water immiscible fluorinated ionic liquids with PF₆⁻ as anion yielded low and only [bmpyr][PF₆] converted maltose somewhat better (25.1 %). Anion variation using EtSO₄⁻, BF₄⁻ and NTf₂⁻ hardly improved maltose conversions. Even though all ionic liquids provide higher polarity, it is obvious that solvent polarity does not necessarily lead to higher conversion rates.

To improve reaction rates with ionic liquids, a minimal solvent approach was attempted too. For this purpose the same ionic liquids were used as coatings on immobilized *Candida antarctica* lipase B and subjected to non solvent conditions, *Method C*. Ionic liquid coated immobilized lipases outperformed ionic liquid solvent conditions.

### Biotransformation in binary solvents

Binary solvents improved maltose conversions and tended to double the efficiency in comparison to mono solvent use (*Method D*). The binary solvent approach is particularly appropriate for hardly miscible substrates such as maltose and linoleic acid, whose solute properties differ considerably. To examine the binary solvent effect more closely, well performing DMF, acetone, THF, acetonitrile, [emim][MeSO₃] including [bmpyr][PF₆] were combined as 1:1 mixtures (*Method D*). DMF/acetone enabled maltose conversion of up to 72.1 % (Table 1). The best green solvent variant acetone/[emim][MeSO₃] yielded 63.6% and the acetone/[bmpyr][PF₆] 60.9%. For the 1:1 ionic liquid mixture,
[emim][MeSO₃]/[bmpyr][PF₆], improved maltose conversion up to 60 %. It has to be taken into account that the combination of two ionic liquids results in a quartenary solvent mixture, [bmpyr][emim][MeS0₃][PF₆]. "Binary ionic liquids" are in this respect rather different from binary organic solvent mixtures. Ionic liquids also offer a very wide variability, which exceeds the possibilities of organic solvents. The work up from binary solutions demands more preparative work than mono solvent use and consequently the eco-efficiency is comparable to ionic liquid use.

**Table 1: Contribution of the solvent to the conversion yield of the reaction**

| **Method** | **Solvent** | **Conversion yield (%)** |
|---|---|---|
| A | Acetonitrile | 29.7 |
| A | THF | 33.9 |
| A | Acetone | 38.1 |
| A | DMF | 39.7 |
| B | [mpim][PF₆] | 19.7 |
| B | [bmpyr][PF₆] | 25.1 |
| B | [emim][MeSO₃] | 36.8 |
| C | DMF / [emim][MeSO₃] | 59.4 |
| C | [bmpyr][PF₆]/ [emim][MeSO₃] | 60.0 |
| C | Acetone / [bmpyr][PF₆] | 60.9 |
| C | DMF / [bmpyr][PF₆] | 63.2 |
| C | Acetone / [emim][MeSO₃] | 63.6 |
| C | THF / [bmpyr][PF₆] | 64.3 |
| C | THF/DMF | 66.2 |
| C | Aceton / DMF | 72.1 |

### Non-solvent parameters

Non-solvent parameters are also decisive for ensuring green biotransformations. This was shown for lipase choice, substrate ratio, molecular sieve content, and reaction time. A series of 10 lipases were subjected to acetone based reaction conditions (*Method A*) (Table 2). As most native lipases exercise their optimal activity between 30-50°C, the enzyme screening was conducted at 40°C. The lowest conversion was provided by *R. arrhizus* with 44.6 % and the best activity was shown by *Pseudomonas cepacia* at 58 %. The immobilized *Candida antarctica* performed among the best with 57.1 %.

**Table 2: Selection of the most efficient enzyme**

| **Enzyme source** | **Units/mg** | **Conversion (%)** |
|---|---|---|
| *R. arrhizuss* | 200 | 44.6 |
| *R. oryzae* | 200 | 46.5 |
| *A. oryzae* | 200 | 47.3 |
| *M. javanicus* | 200 | 48.8 |
| *C. rugosa* | 200 | 52.8 |
| CAL-B (*C*. *antartica*) | 200 | 53 |
| *C. cylindracea* | 200 | 54.1 |
| *P. fluorescens* | 200 | 54.3 |
| CAL-B immobilized (*C*. *antartica*) | 200 | 57.3 |
| *P. cepacia* | 200 | 58 |

Enzyme quantities also influenced substrate conversions although maltose does not dissolve very well in acetone. In contrast, an excess of linoleic acid of four or six equivalents improved maltose conversions only up to 10%. However, water removal with molecular sieves 3Å correlated well with maltose conversions. Finally, prolonging the process to 7 days yielded a maltose conversion of up to 82%. In conclusion solvent choice is a crucial parameter in eco-efficient mono-linoleyl-α-D-maltose synthesis. In addition, process time, enzyme choice, and water removal are influential non-solvent parameters that potentially enhance the eco-efficiency of a lipase mediated acylation.

### Regioisomer analysis

The disaccharide fatty acid ester analysis is challenging as a mixture of regioisomers was obtained. NMR spectroscopy is the method of choice in the structure elucidation of regioselectively acylated maltose. Analytical HPLC separated the isolated product mixture into two isomers, which eluted at 7.77 and 8.14 min in a 54.8 % to 45.2 % ratio. An electro spray mass spectrum showed a single molecular mass peak at 604 m/z confirming the presence of two structural isomers. A ¹³C-¹H-NMR correlation (HSQC) permitted to distinguish the maltose hydroxyl protons from carbon linked protons as no correlation with carbon atoms is possible. A high field 800 MHz spectrometer substantially reduced the signal overlapping of the hydroxyl protons. The ¹H-NMR spectrum confirmed the presence of two regioisomers visible as two doublets OH-(C1) at δ_{H} = 6.81 and 6.42 ppm. Coincidentally, they have the same coupling constant *J*_{OH,1} = 6.32 Hz, but do not couple. From the well resolved hydroxyl protons HO-C(1,2',3) the isomer ratio was calculated as 58.2% for 6'-O-linoleyl-α-D-maltose and 41.8 % for 6-O-linoleyl-α-D-maltose. This corresponds to the isomer ratio found with HPLC. The non-acylated triplet hydroxyls HO-C(6,6') at δ_{OH} = 4.62 and 4.52 ppm also show a 58:42 % ratio, which is again in line with the isomer ratio found for the secondary hydroxyl groups, HO-C(1,2',3). Most importantly, it shows that no double acylation occurred.

### Conclusions

Solvent choice is an important parameter in eco-efficient lipase catalysis of mono-linoleyl-α-D-maltose. Mono solvent conditions, using acetone, DMF, THF, acetonitrile and the ionic liquid [emim][MeSO₃], enabled fair to good maltose conversions (82%). Acetone and [emim][MeSO₃] excelled as green solvents and are equally well suited as mineral oil based organic solvents. Binary green solvents, [emim][MeSO₃]/[bmpyr][PF₆] and the acetone/[emim][MeSO₃] mixture tended to double maltose conversions.

From an industrial point of view, the best solvent is acetone. Unlike ionic liquids and binary mixtures, it is easily removable. Moreover, acetone is to some extent green as it can be produced from renewable feedstock.

## Claims

1. Green process for the lipase mediated esterification of maltose or a maltose oligomer with a medium chain polyunsaturated fatty acid, wherein said process comprising the steps of:
(a) incubating a medium chain polyunsaturated fatty acid and maltose or a maltose oligomer in the presence of a lipase, a binary solvent and an activated molecular sieve 3Å in a sealed container under agitation for 2 to 7 days at a temperature of 30 to 80°C,
(b) filtering off the lipase and activated molecular sieve,
wherein the binary solvent primarily consists of any mixture of 2 solvents selected from green organic solvents and ionic liquids.

2. A process according to claim 1, wherein the lipase is selected from *P. cepia, C. antartica,* and immobilized *C*. *antartica* lipase.

3. A process according to claim 1 or 2, wherein the lipase is added to the reaction mixture at concentration comprised between 1000 and 3000 units lipase per mmol oligosaccharide.

4. A process according to any of claims 1 to 3, wherein the maltose oligomer is selected from maltotriose, maltotetrose, and maltopentose.

5. A process according to any of the claims 1 to 3, wherein maltose is used.

6. A process according to any of the claims 1 to 5, wherein the medium chain polyunsaturated fatty acid is any of C₁₄ to C₂₂ polyunsaturated fatty acids.

7. A process according to any of the claims 1 to 5, wherein the medium chain polyunsaturated fatty acid is any of C₁₆ to C₂₀ polyunsaturated fatty acids.

8. A process according to any of the claims 1 to 5, wherein the medium chain polyunsaturated fatty acid is linoleic acid.

9. A process according to any of the claims 1 to 8, wherein the binary solvent primarily consists of any mixture of 2 solvents selected from: acetone, acetonitrile, dimethylformamide, tetrahydrofurane, 1-ethyl-3-methylimidazolium methanesulphonate, 1-methyl-3-pentyl imidazolium hexafluorophosphate, and 1-butyl-1-methylpyrrolidinium hexafluorophosphate.

10. A process according to any of the claims 1 to 9, wherein the binary solvent is selected from tetrahydrofurane/dimethylformamide, acetone/dimethylformamide, tetrahydrofurane/1-butyl-1-methylpyrrolidinium hexafluorophosphate, acetone/1-ethyl-3-methylimidazolium methanesulphonate, dimethylformamide/1-butyl-l-methylpyrrolidinium hexafluorophosphate, and acetone/1-butyl-1-methylpyrrolidinium hexafluorophosphate.

11. A process according to claim 10, wherein the ratio of the two solvents is between 1:5 and 5:1.

12. A process according to any of the claims 1 to 11, wherein the step a) is performed from 2 to 4 days at a temperature of 40 to 70°C.

13. A process according to any of the claims 1 to 12, wherein the molar ratio maltose or maltose oligomer/medium chain polyunsaturated fatty acid is comprised between 0.1 and 1.

14. A process according to any of the claims 1 to 13, wherein molar ratio maltose or maltose oligomer/medium chain polyunsaturated fatty acid is comprised between 0.4 and 0.8.

15. A process according to any of the claims 1 to 14, wherein the molecular sieve is added to the reaction mixture at concentration comprised between 15 and 300 g/litre.

16. Use of the process according any of the claims 1 to 15, for the production of mono-linoleyl-α-D-Maltose with a ratio 6'-O-linoleyl-α-D-maltose: 6-O-linoleyl-α-D-maltose comprised between 62:38 and 54:46
